# EUROPEAN PATENT SPECIFICATION

(11) **EP 3 596 120 B1**
(45) Date of publication and mention of the grant of the patent: **13.12.2023**
(21) Application number: 18713147.9
(22) Date of filing: 14.03.2018
(51) Int. Cl.: C07K 16/18

(54) **COLLAGEN TYPE XVIII ASSAY**
KOLLAGEN-TYP-XVIII-TEST
DOSAGE DE COLLAGÈNE DE TYPE XVIII

(30) Priority: 16.03.2017 GB 201704182
(43) Date of publication of application: 22.01.2020
(73) Proprietor: Nordic Bioscience A/S, 2730 Herlev (DK)
(72) Inventor: MANON-JENSEN, Tina, DK-2605 Brondby (DK); KARSDAL, Morten, DK-2100 København Ø (DK)
(74) Representative: Beck Greener LLP
(86) International application number: PCT/EP2018/056320
(87) International publication number: WO 2018/167111

(56) References cited:
- JANNA SAARELA ET AL: "The Short and Long Forms of Type XVIII Collagen Show Clear Tissue Specificities in Their Expression and Location in Basement Membrane Zones in Humans", AMERICAN JOURNAL OF PATHOLOGY., vol. 153, no. 2, 1 August 1998 (1998-08-01), pages 611-626, XP55472990, US ISSN: 0002-9440, DOI: 10.1016/S0002-9440(10)65603-9
- ELAMAA HARRI ET AL: "Characterization of the human type XVIII collagen gene and proteolytic processing and tissue location of the variant containing a frizzled motif", MATRIX BIOLOGY, ELSEVIER, NL, vol. 22, no. 5, 1 September 2003 (2003-09-01), pages 427-442, XP002477574, ISSN: 0945-053X, DOI: 10.1016/S0945-053X(03)00073-8
- LOTTA SEPPINEN ET AL: "The multiple functions of collagen XVIII in development and disease", MATRIX BIOLOGY, ELSEVIER, NL, vol. 30, no. 2, 22 November 2010 (2010-11-22), pages 83-92, XP028169413, ISSN: 0945-053X, DOI: 10.1016/J.MATBIO.2010.11.001 [retrieved on 2010-12-14]
- BAGER C L ET AL: "Type XVIII Collagen", BIOCHEMISTRY OF COLLAGENS, LAMININS AND ELASTIN: STRUCTURE, FUNCTION AND BIOMARKERS 2016 ACADEMIC PRESS LTD-ELSEVIER SCIENCE LTD, 24-28 OVAL ROAD, LONDON NW1 7DX, UK, BIOCHEMISTRY OF COLLAGENS, LAMININS AND ELASTIN: STRUCTURE, FUNCTION AND BIOMARKERS, 1 January 2016 (2016-01-01), pages 113-121, XP009505174, DOI: 10.1016/B978-0-12-809847-9.00018-0 ISBN: 978-0-12-809847-9
- Nadja Gad Kjeld ET AL: "The endothelial specific isoform of type XVIII collagen correlates to annual bleeding rate in haemophilia patients", PLoS One, 1 January 2018 (2018-01-01), page e0190375, XP055472393, San Francisco DOI: 10.1371/journal.pone.0190375 Retrieved from the Internet: URL:http://journals.plos.org/plosone/artic le/file?id=10.1371/journal.pone.0190375&ty pe=printable

## Description

### Field of the Invention

The present invention relates to a method for detecting the short isoform of Collagen type XVIII, and the use of said method in evaluating haemophilic diseases.

### Background

Recurrent haemarthroses due to vascular ruptures is a major complication in haemophilia, contributing to progressive joint damage, which leads to haemophilic (HF) arthropathy. The medical need in the HF field to reduce bleeding incidents requires measurement of the annual bleeding rate (ABR) in haemophiliacs. Although a crude measure, the ABR is associated with HF arthropathy [1] but is also a key parameter in clinical trials, ensuring quantifiable benefits to patients [2-4]. Endothelial cell impairment and matrix quality may be associated with joint bleeds and later the development of HF arthropathy.

Vascular rupture is associated with the quality and turnover of the basement membrane (BM) located directly underneath the endothelial cells. Extracellular matrix turnover is a central pathological feature in many diseases due to epithelial or endothelial cell damage. While the endothelial cell function is debated, no quantifiable methods are available for specifically quantifying the damage to the vascular endothelium, which, subsequent to bleeding, results in exposure of the BM underlying the endothelial cells.

Quantifying the BM proteins specific to the endothelial cells may therefore have particular relevance to endothelial cell stability and rupture in haemophiliacs.

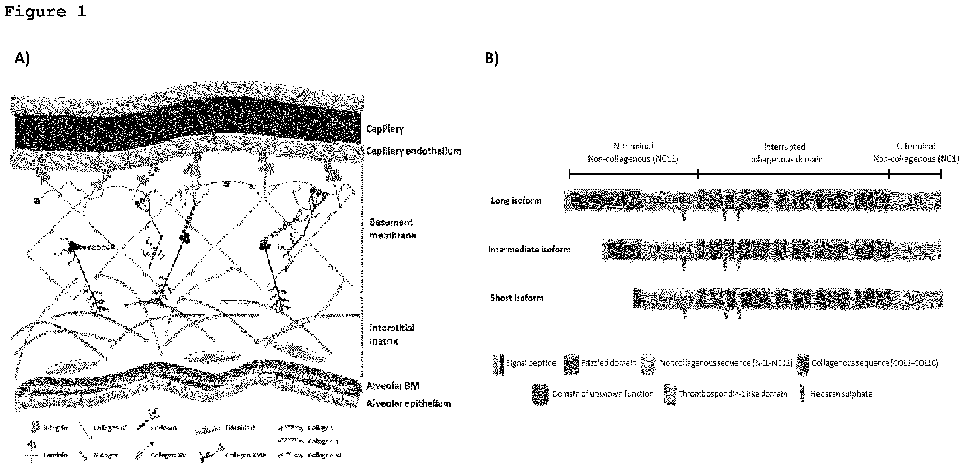

Collagen IV, XV and XVIII represent the most well-known collagens of the vascular BMs, responsible for maintaining vessel wall structure and integrity of the membrane (Fig.1A) [5-7].

Type XVIII collagen exists in three isoforms: short, intermediate, and long, localized in various basement membrane zones [4-6] (Fig.1B). All three isoforms contain a thrombospondin 1-like domain and 10 triple helical collagenous domains (Col1-10) flanked by 11 noncollagenous domains (NC1-11). The NC1 domain contains a C-terminal endostatin domain that has antiangiogenic properties [7]. The short isoform is endothelial specific and is found in blood vessels and around muscular structures. Here, zero or only very low amounts of the intermediate and long isoforms are present [5]. Following remodelling, damage, and degradation of the vascular BM the short isoform of collagen type XVIII may be affected and degraded, releasing measurable fragments of type XVIII collagen, as have been undertaken with other types of collagen [8,9].

Mutations in type XVIII collagen have also been linked to the autosomal recessive disorder Knobloch syndrome (KS). KS is characterized by various eye defects leading to blindness at a young age [11,12]. Moreover, col18a1-/- knockout mice showed delayed regression of blood vessels in the vitreous along the surface of the retina, impaired angiogenesis of retinal vessels and altered iris BM structure [8,13-16]. Thus, collagen XVIII is essential for controlling blood vessel formation in the eye, and possibly an important component in the BM zones of the entire vascular system [17].

It is known that the three isoforms of collagen type XVIII differ in their N-terminal sequences [31-34]. Various authors described the creation of antibodies that target all three isoforms or one or both of the intermediate and long isoforms [31-33].

The present Applicant is unaware of any biomarkers and/or antibodies specific to the short isotype of type XVIII collagen. The majority of commercially available antibodies recognize the C-terminal endostatin end of type XVIII collagen hence it is not possible to differentiate between the three isotypes using those antibodies.

Thus, there is a need for antibodies and/or biomarkers measuring the specific short isotype of type XVIII collagen and excluding the two other isoforms to quantify vascular specific basement membrane turnover in terms of detection of endothelial type XVIII collagen content.

### Summary of the Invention

The Applicant has now developed an assay for detecting the short isoform of collagen type XVIII, and has used that assay to assess the clinical relevance of turnover of collagen type XVIII in patients diagnosed with HF arthropathy.

In a first aspect, the invention relates to an antibody specifically reactive with short isoform collagen type XVIII, wherein said antibody does not react with intermediate isoform collagen type XVIII or with long isoform collagen type XVIII, wherein the antibody is specifically reactive with the N-terminal amino acid sequence of H₂N-EPERISEEVG (SEQ ID NO: 1), and wherein the antibody does not specifically recognise or bind an N-extended elongated version of said N-terminal amino acid sequence which is H₂N-AEPERISEEVG (SEQ ID NO: 3) and/or does not specifically recognise or bind an N-truncated version of said N-terminal amino acid sequence which is H₂N-PERISEEVG (SEQ ID NO: 4). Accordingly, the antibody is specifically reactive with an N-terminal epitope of short isoform collagen type XVIII that is exposed after cleavage and removal of the N-terminal signal peptide of short isoform collagen type XVIII.

The antibody may be a monoclonal or polyclonal antibody. Preferably, the antibody is a monoclonal antibody.

In a second aspect, the invention relates to a method of immunoassay for detecting or quantitating in a sample short isoform collagen type XVIII, wherein said method comprises contacting a sample comprising said short isoform collagen type XVIII with an antibody as described supra, and determining the amount of binding of said antibody.

The present invention may be directed to a method of detecting short isoform collagen type XVIII in a human patient, said method comprising:
a. obtaining a sample from the human patient; and
b. detecting whether said short isoform collagen type XVIII is present in the sample by contacting the sample with an antibody (as described supra) and detecting binding between short isoform collagen type XVIII and the antibody.

Preferably, the sample is a biofluid. The biofluid may be, but is not limited to, serum, plasma, urine, cerebrospinal fluid, or amniotic fluid.

The immunoassay may be a competition assay or a sandwich assay. The immunoassay may be a radioimmunoassay or an enzyme-linked immunosorbent assay.

The method may further comprise correlating the quantity of said short isoform collagen type XVIII determined by said method with standard haemophilic disease samples of known disease severity to evaluate the severity of a haemophilic disease. In this regard, "standard haemophilic disease samples" means samples obtained from subjects known to have a haemophilic disease of a known severity.

Alternatively, or in addition to, the method may comprise comparing the quantity of said short isoform collagen type XVIII determined by said method with standard values associated with healthy subjects to evaluate the presence and/or severity of a haemophilic disease. In this regard "standard values associated with healthy subjects" means standardised quantities of short isoform collagen type XVIII determined by the method described supra for subjects considered to be healthy, i.e. without a haemophilic disease. The standardisation will depend on the height, weight, gender, etc. of the healthy subject.

Alternatively, or in addition to, the method may further comprise quantifying the amount of collagen type XVIII in at least two samples obtained from a subject at a first time point and at at least one subsequent time point, wherein an increase in the quantity of collagen type XVIII from the first time point to the at least one subsequent time point is indicative of a deterioration in a haemophilic disease from the first time point to the at least one subsequent time point, or wherein a decrease in the quantity of collagen type XVIII from the first time point to the at least one subsequent time point is indicative of an improvement in a haemophilic disease from the first time point to the at least one subsequent time point.

The haemophilic disease may be haemophilic arthropathy. The method described supra may also be used to evaluate Knobloch syndrome.

In another aspect, the present invention is directed to a method for evaluating the efficacy of a drug for treating a haemophilic disease. The method comprises using the method as described above to quantify the amount of collagen type XVIII in at least two biological samples obtained from a subject at a first time point and at at least one subsequent time point during a period of administration of the drug to the subject. A reduction in the quantity of collagen type XVIII from the first time point to the at least one subsequent time point during the period of administration of the drug is indicative of an efficacious drug for treating a haemophilic disease.

In a final aspect, the invention relates to an assay kit for determining the quantity of short isoform collagen type XVIII, comprising an antibody as described supra and at least one of:
- a streptavidin coated 96 well plate
- a peptide which is reactive with said antibody, which may be a biotinylated peptide H₂N-EPERISEEVG-L-Biotin (SEQ ID NO: 5), wherein L is an optional linker
- an optionally biotinylated secondary antibody for use in a sandwich immunoassay
- a calibrator peptide comprising the N-terminal sequence H₂N-EPERISEEVG (SEQ ID NO: 6)
- an antibody HRP labelling kit
- an antibody radiolabeling kit
- an assay visualization kit.

### Figures

**Figure 1A****.** Structure of the vascular BM. The capillary subendothelial layer is composed of a BM and an interstitial matrix (IM). The main components of vascular BMs include type IV collagen, laminin and nidogen. Minor components include type XV collagen and type XVIII collagen. The components of the BM self-assemble into sheet-like structures. The BM is tightly connected to the IM through interactions between collagen type I and VI and collagen type IV and XV.
**Figure 1B****.** Isoforms of Type XVIII collagen. Collagen type XVIII exists in three isoforms, which differ in their N-terminus. The *COL18A1* gene encodes these variants by the use of two promoters and alternative splicing. The short isoform has a different signal peptide from the other two, and is coded by promoter 1, while the others have the same signal peptide and are both coded by promoter 2. All isoforms include a thrombospondin-like domain, heparin sulphate chains and a globular C-terminal containing the type XVIII collagen fragment, endostatin.

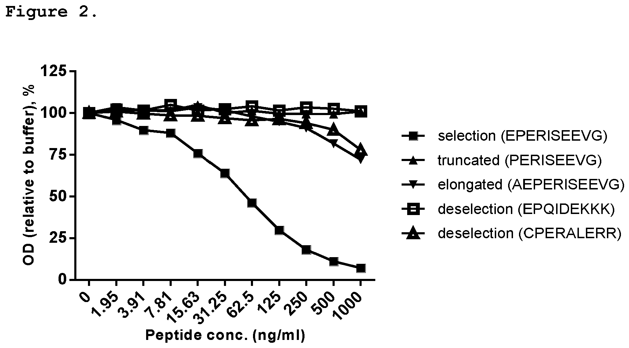

**Figure 2****.** COL-18N antibody specific reactivity against type XVIII collagen. Monoclonal NB632-13H11/G5 antibody reaction towards standard peptide, truncated, elongated and de-selection peptides is shown. The antibody has high reactivity towards standard peptide and no or minimal cross-reactivity with the other peptides.

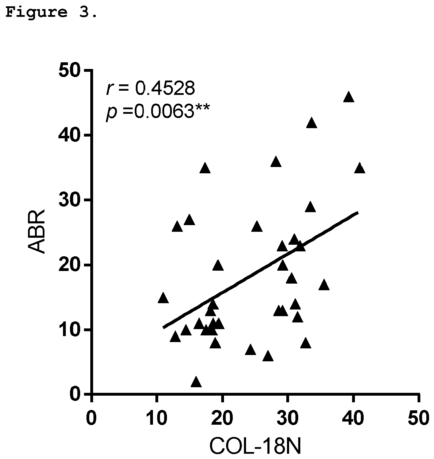

**Figure 3****.** COL-18N correlation with ABR. Serum from 35 male HF patients aged 26 and over was measured with the COL-18N ELISA. Correlations between vascular endothelial type XVIII collagen concentration and ABR were analyzed using Spearman rank correlation coefficient and shown r=0.45, p<0.006. Differences between ABR and COL-18N levels were considered statistically significant if p<0.05 and significant levels are displayed as: *=p<0.05; **=p<0.01, and ***=p<0.001.

### Definitions

As used herein the term "N-terminal epitope" refers to an N-terminal peptide sequence at the extremity of a polypeptide, i.e. at the N-terminal end of the polypeptide, and is not to be construed as meaning in the general direction thereof.

As used herein the term, the term "competitive ELISA" refers to a competitive enzyme-linked immunosorbent assay and is a technique known to the person skilled in the art.

As used herein the term "sandwich immunoassay" refers to the use of at least two antibodies for the detection of an antigen in a sample, and is a technique known to the person skilled in the art.

As used herein, the term "short isoform of collagen type XVIII" refers to the isoform of collagen type XVIII generated by promoter 1 wherein the N-terminal non-collagenous region includes the thrombospondin-1 like domain (TSP-1), but does not contain the Domain of Unknown Function (DUF) or the Frizzled Domain (FZ). The term "intermediate isoform of collagen type XVIII" refers to the alternatively spliced isoform of collagen type XVIII generated by promoter 2 wherein the N-terminal non-collagenous region includes the TSP-1 and DUF, but does not comprise the FZ. The term "long isoform of collagen type XVIII" refers to the alternatively spliced isoform of collagen type XVIII generated by promoter 2 wherein the N-terminal non-collagenous region includes the TSP-1, the DUF and the FZ.

As used herein the term, the term "COL-18N" is used as shorthand to describe the herein disclosed specific assay for the N-terminal sequence EPERISEEVG (SEQ ID NO: 1) of the short isoform of collagen type XVIII.

### Examples

The presently disclosed embodiments is described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Patient samples

Serum was collected from 35 male HF patients aged 26 and over. This cut-off age was chosen as collagen turnover wears off at the closure of the growth plate at the age of approximately 25 years [21]. The patients had a treatment history of either on-demand medication upon bleeding episodes or intake of a low dosage of prophylaxis of 5-10 IU/kg recombinant FVIII, 2-3 times/week. Patients had varying degrees of HF arthropathy defined by the World Federation of Haemophilia Physical Examination Score (Gilbert Score) and by radiologic evaluation according to the Pettersson score. The patients' average ABR was 18.1 ranging from 2-46. Exclusion criteria were bleeding disorders other than haemophilia, human immunodeficiency virus infection, chronic obstructive pulmonary disease, medical history of joint disease or liver fibrosis, and treatment with anti-inflammatory biologics or steroids. Study participants were enrolled at the Department of Haematology, Peking Union Medical College Hospital, Beijing, China. The study was approved by the Peking Union Medical College Hospital, Chinese Academy of Medical Sciences, Ethics Review Board, with the serial number S-720. Signed informed consent was obtained from all subjects.

### Monoclonal antibody development for COL-18N

A peptide corresponding to the first 10 amino acids of the N-terminal epitope of the short isoform of human type XVIII collagen α1 chain (excluding signal peptide, ³⁴'EPERISEEVG'⁴³) was used to generate monoclonal neo-epitope specific antibodies. Beijing Administration Office of Laboratory Animal and animal ethics committee of Nordic Bioscience approved the animal work. Generation of monoclonal antibodies was initiated by subcutaneous immunization of 6-8 week old Balb/C mice using 200µL emulsified Freund's complete adjuvant with 60µg peptide conjugated to keyhole limpet hemocyanin (KLH). Consecutive immunizations were preformed at 2-week intervals in Freund's incomplete adjuvant, until stable titer levels were reached. The mouse was boosted intravenously with 50ug immunogen in 100µL 0.9% sodium chloride solution and three days later the spleen cells were fused with SP2/0 myeloma cells (LGC Standards AB, Boras, Sweden) [22]. The hybridomas were grown in 96-well plates and monoclonal growth was ensured by limited dilution. Clones were screened against the specific epitope (EPERISEEVG; SEQ ID NO: 1), elongated peptide (AEPERISEEVG; SEQ ID NO: 3) and truncated peptide (PERISEEVG; SEQ ID NO: 4). The mAb producing clone, NB632-13H11/G5, was selected based on reactivity to above-mentioned peptides, and antibody purified using Protein G columns (GE Healthcare, Hilleroed, Denmark).

### COL-18N ELISA protocol

The competitive COL-18N ELISA was performed as follows. A 96-well streptavidin-coated plate (Roche cat.: 11940279) was coated with 100µl/well 1.25ng/mL biotinylated synthetic peptide EPERISEEVG-K-Biotin (SEQ ID NO: 7) dissolved in coating buffer (20mM Na₂HPO₄, 3.7mM KH₂PO₄, 137mM NaCl, 2.7mM KCL, 0.1% Tween20, 1% BSA, pH7.4) and incubated for 30 min. at 20°C. The plate was washed five times with washing buffer (20mM Tris, 50mM NaCl, pH 7.2). 20µL of the standard peptide (EPERISEEVG; SEQ ID NO: 6) or samples diluted in incubation buffer (20mM Na₂HPO₄, 3.7mM KH₂PO₄, 137mM NaCl, 2.7mM KCL, 0.1% Tween20, 1% BSA, 5% Liquid II pH7.4) were added to appropriate wells, followed by 100uL/well monoclonal antibody NB632-13H11/G5, and incubated for 1hr at 20°C. After washing, 100µl rabbit-anti-mouse antibody (Jackson, 315-035-045) was added 1:3000 dissolved in coating buffer and incubated 1hr at 20°C, 300rpm. After final five times wash, the wells were incubated with 100uL tetramethylbenzidine (TMB) (Kem-En-Tec cat. 438OH) at 20°C, 300rpm in the dark for 15 min., followed by the addition of 100pL/well-stopping solution (1% H₂SO₄). The colorimetric reaction was measured at 450nm with 650nm as reference, and a calibration curve was plotted using a 4-parametric mathematical fit model.

### COL-18N technical evaluation

Technical assay validation was performed according to international guidelines. The lower limit of detection (LLOD) was calculated as mean + 3x standard deviation (SD) determined from 21 zero samples (i.e., the assay buffer). The upper limit of detection (ULOD) was determined as the mean - 3xSD of 10 measurements of standard A (1000ng/ml). The lower limit of quantification (LLOQ) was determined by the lowest possible concentration with an imprecision of less than 30%. The intra- and inter-assay variations were calculated as the mean of the variation of seven human samples by 10 independent runs in duplicates. Dilution recovery was determined in a 2-fold dilution of two human serum and three human citrate plasma, calculated as percentage recovery of diluted matrices compared to undiluted ones. Spiking recovery was assessed in human serum and citrate plasma spiked with standard peptide at concentrations covering the entire measure range or by combining two samples of similar concentration in order to double the concentration. Spiking recovery was calculated as the measured amount percentage recovery of the theoretical amount. Interference by hemoglobin, lipemia, biotin, and human antibodies against mouse antigens by human anti-mouse antibody (HAMA) was determined by adding two-fold dilutions to a serum sample of known concentration. Concentrations started at 0.500 mmol/l hemoglobin, 0.56 mmol/l lipemia, 160 pg/l biotin and 2010ng/ml HAMA. Recovery percentage was calculated with the normal serum sample as a reference value. Analyte stability was determined for two healthy human serum samples and one healthy citrate plasma sample for four freeze-thaw cycles and calculated as the percentage recovery of the first freeze-thaw cycle. Same samples were tested at 2 hrs, 4 hrs and 24 hrs at 4°C and 20°C against non-stressed analytes. Finally, antibody specificity was assessed by a sanity check testing reactivity towards standard (EPERISEEVG; SEQ ID NO: 1), elongated (AEPERISEEVG; SEQ ID NO: 3), truncated (PERISEEVG; SEQ ID NO: 4) and de-selection peptides (EPQIDEKKK (SEQ ID NO: 8) and CPERALERR (SEQ ID NO: 9)).

### Statistics

Correlations between serum COL18-N concentration and ABR were analyzed using Spearman rank correlation coefficient with GraphPad Prism v6 (GraphPad Software, La Jolla, CA, USA). Differences were considered statistically significant if p<0.05.

### RESULTS AND DISCUSSION

A novel competitive ELISA using a monoclonal antibody to detect COL-18N in human serum and plasma (citrate, EDTA, heparin) samples was developed and evauated.

The main findings were:
- Serum COL-18N levels were correlated with ABR in HF patients.
- A technically stable assay for detecting COL-18N in human serum and human plasma with acceptable intra-inter assay variations and acceptable dilution and spike recoveries.

### Characterization of COL-18N ELISA

A competitive COL-18N ELISA that can assess endothelial BM degradation was developed. The technical performance of the ELISA is summarized in table 1, providing a measurement range from 4.8-671ng/ml, intra- and inter-variability at 7% and 13% respectively, dilution and spike recovery within 100120%, and analytic stability with no immunoassay interference. The normal concentration of COL-18N in serum (16.6ng/ml), plasma citrate (12.5ng/ml), EDTA plasma (13.2ng/ml), and heparin plasma (15.8ng/ml) was consistent regardless of matrices.

The NB632-13H11/G5 antibody specially recognized the first 10 amino acids of N-terminus type XVIII collagen α1 chain, short isoform (selection) (Fig. 2). The antibody showed no or minimal reactivity towards related peptides, indicating a high specificity (Fig. 2). Possible cross-reactivity with N-terminus of intermediary and long forms of collagen type XVIII is not plausible. The three isoforms of collagen type XVIII are encoded by the *COL18A1* gene by the use of two different promoters and alternative splicing (18,19) (Fig.1B). As a result N-terminus of the short isoform becomes entirely different from N-terminus of the other two isoforms.

The technical evaluation of the competitive COL-18N ELISA revealed a stable sensitive assay with high specificity towards the N-terminus of vascular form of type XVIII collagen including high accuracy and precision of the assay.

**Table 1. Technical performance of COL-18N ELISA.**

| **Measurements** | **Technical characteristics** |
|---|---|
| Lower limit of detection | 4.8 ng/ml |
| Upper limit of detection | 671 ng/ml |
| Lower limit of quantification | 7.3 ng/ml |
| Intra-assay variability | 7% (accepted <10%) |
| Inter-assay variability | 13% (accepted <15%) |
| Dilution recovery | within 100 ± 20% |
| Spiking recovery | within 100 ± 20% |
| Freeze-thaw stability (4 cycles) | within 100 ± 20% |
| Analyte stability (0-20 hrs at 4°C and 20°C) | within 100 ± 20% |
| Interference (Hgb, lipid, Biotin, HAMA) | No interference |
| Human healthy serum (n=10, mean value) | 16.6 ng/ml |
| Human healthy plasma citrate (n=10, mean value) | 12.5 ng/ml |
| Human healthy plasma EDTA (n=10, mean value) | 13.2 ng/ml |
| Human healthy plasma heparin (n=10, mean value) | 15.8 ng/ml |

COL-18N correlates to annual bleeding rate in HF patients. The haemorrhagic disorder haemophilia manifests clinically by repeated haemarthrosis resulting in unavoidable arthropathy in the absence of adequate treatment. One cardinal feature of medicinal intervention in haemophilia is lowering of ABR, albeit objective quantifiable parameters with high resolution are lacking.

The bleeding severity of haemophilia is generally inversely proportional to the degree of FVIII/IX activity in the plasma, although substantial variability in bleeding tendencies is well-known. Reduced spontaneous bleeding and lower requirements of factor concentrates are reported in a subset of 10-15% of severe HF patients [23,24]. Also, development of inhibitors in non-severe HF patients may heighten the bleeding phenotype considerably [25].

Bleeding phenotype may be further compromised by large discrepancies amongst the FVIII assays caused by standardization of the assays [26] and may even be influenced by the type of FVIII concentrates used during therapy [27,28]. Other assays, like thrombin generation, correlates to the bleeding phenotype in HF patients [29], but is inconsistent in HF patients with FVIII inhibitors despite the occurrence of thrombin generation [30].

In haemophilia, consequent to *i*) endothelial cell damage *ii*) bleeding and *iii*) delayed clotting and wound healing, the endothelial remodelling contributing to clinical symptoms of haemophilia and pathophysiological disease representation may be affected. It has now been found using the herein described assay that vascular endothelial type XVIII collagen correlates with ABR in HF patients (Fig.3, r=0.45, p<0.006). Objective biomarkers of pathological processes, like those of degraded type XVIII collagen that associates with ABR, may assist in benchmarking treatments, monitoring patients and consequently may assist in drug development for the benefit of patients.

### CONCLUSION

In summary, the data combined suggests that the technically robust COL-18N biomarker can be related to pathologies involving vascular BM degradation and remodelling, which affects degradation of the short isoform of type XVIII collagen. In addition, the data enables the COL-18N biomarker to evaluate ABR for optimal treatment and monitoring of patients to prevent the development of arthropathy.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. All prior teachings acknowledged above are hereby incorporated by reference. No acknowledgement of any prior published document herein should be taken to be an admission or representation that the teaching thereof was common general knowledge in Australia or elsewhere at the date hereof.

### REFERENCES

1 Funk MB, Schmidt H, Becker S, Escuriola C, Klarmann D, Klingebiel T, Kreuz W. Modified magnetic resonance imaging score compared with orthopaedic and radiological scores for the evaluation of haemophilic arthropathy. Haemophilia 2002; 8: 98-103.
2 Fischer K, Steen Carlsson K, Petrini P, Holmstrom M, Ljung R, van den Berg HM, Berntorp E. Intermediate-dose versus high-dose prophylaxis for severe hemophilia: comparing outcome and costs since the 1970s. Blood 2013; 122: 1129-36.
3 Gringeri a, Lundin B, von Mackensen S, Mantovani L, Mannucci PM. A randomized clinical trial of prophylaxis in children with hemophilia A (the ESPRIT Study). J Thromb Haemost 2011; 9: 700-10.
4 Lundin B, Ljung R, Pettersson H. MRI scores of ankle joints in children with haemophilia - comparison with clinical data. Haemophilia 2005; 11: 116-22.
5 Tomono Y, Naito I, Ando K, Yonezawa T, Sado Y, Hirakawa S, Arata J, Okigaki T, Ninomiya Y. Epitope-defined monoclonal antibodies against multiplexin collagens demonstrate that type XV and XVIII collagens are expressed in specialized basement membranes. Cell Struct Funct 2002; 27: 9-20.
6 Ricard-Blum S, Ruggiero F. The collagen superfamily: from the extracellular matrix to the cell membrane. Pathol Biol 2005; 53: 430-42.
7 Manon-Jensen T, Kjeld NG, Karsdal MA. Collagen-mediated hemostasis. J Thromb Haemost 2016; 14: 438-48.
8 Elamaa H, Snellman A, Rehn M, Autio-Harmainen H, Pihlajaniemi T. Characterization of the human type XVIII collagen gene and proteolytic processing and tissue location of the variant containing a frizzled motif. Matrix Biol 2003; 22: 427-42.
9 Saarela J, Rehn M, Oikarinen a, Autio-Harmainen H, Pihlajaniemi T. The short and long forms of type XVIII collagen show clear tissue specificities in their expression and location in basement membrane zones in humans. Am J Pathol American Society for Investigative Pathology; 1998; 153: 611-26.
10 Seppinen L, Pihlajaniemi T. The multiple functions of collagen XVIII in development and disease. Matrix Biol International Society of Matrix Biology; 2011; 30: 83-92.
11 Marneros AG, Olsen BR. Physiological role of collagen XVIII and endostatin. FASEB J 2005; 19: 716-28.
12 Passos-Bueno MR, Suzuki OT, Armelin-Correa LM, Sertié AL, Errera FI V, Bagatini K, Kok F, Leite KRM. Mutations in collagen 18A1 (COL18A1) and their relevance to the human phenotype. An Acad Bras Cienc 2006; 78: 123-31.
13 Marneros AG, Keene DR, Hansen U, Fukai N, Moulton K, Goletz PL, Moiseyev G, Pawlyk BS, Halfter W, Dong S, Shibata M, Li T, Crouch RK, Bruckner P, Olsen BR. Collagen XVIII/endostatin is essential for vision and retinal pigment epithelial function. EMBO J 2004; 23: 89-99.
14 Utriainen A, Eklund L, Pihlajaniemi and T. Structurally altered basement membranes and hydrocephalus in a type XVIII collagen deficient mouse line. Hum Mol Genet 2004; 13: 2089-99.
15 Marneros AG, Olsen BR. Age-Dependent Iris Abnormalities in Collagen XVIII/Endostatin Deficient Mice with Similarities to Human Pigment Dispersion Syndrome. Investig Opthalmology Vis Sci 2003; 44: 2367.
16 Fukai N. Lack of collagen XVIII/endostatin results in eye abnormalities. EMBO J 2002; 21: 1535-44.
17 Aikio M, Elamaa H, Vicente D, Izzi V, Kaur I, Seppinen L, Speedy HE, Kaminska D, Kuusisto S, Sormunen R, Heljasvaara R, Jones EL, Muilu M, Jauhiainen M, Pihlajamäki J, Savolainen MJ, Shoulders CC, Pihlajaniemi T. Specific collagen XVIII isoforms promote adipose tissue accrual via mechanisms determining adipocyte number and affect fat deposition. Proc Natl Acad Sci U S A 2014; 111: E3043-52.
18 Carmeliet P, Collen D. Molecular Analysis of Blood Vessel Formation and Disease. Am J Physiol 1997; 273: 2091-104.
19 Karsdal MA, Bay-Jensen AC, Leeming DJ, Henriksen K, Christiansen C. Quantification of "end products" of tissue destruction in inflammation may reflect convergence of cytokine and signaling pathways - implications for modern clinical chemistry. Biomarkers 2013; 18: 375-8.
20 Karsdal MA, Nielsen MJ, Sand JM, Henriksen K, Genovese F, Bay-Jensen A-C, Smith V, Adamkewicz JI, Christiansen C, Leeming DJ. Extracellular Matrix Remodeling: The Common Denominator in Connective Tissue Diseases Possibilities for Evaluation and Current Understanding of the Matrix as More Than a Passive Architecture, but a Key Player in Tissue Failure. Assay Drug Dev Technol 2013; 11: 70-92.
21 Iki M, Akiba T, Matsumoto T, Nishino H, Kagamimori S, Kagawa Y, Yoneshima H. Reference database of biochemical markers of bone turnover for the Japanese female population. Japanese Population-based Osteoporosis (JPOS) Study. Osteoporos Int 2004; 15: 981-91.
22 Gefter ML, Margulies DH, Scharff MD. A simple method for polyethylene glycol-promoted hybridization of mouse myeloma cells. Somatic Cell Genet 1977; 3: 231-6.
23 Aledort LM, Haschmeyer RH, Pettersson H. A longitudinal study of orthopaedic outcomes for severe factor-VIII-deficient haemophiliacs. The Orthopaedic Outcome Study Group. J Intern Med 1994; 236: 391-9.
24 Aznar JA, Magallon M, Querol F, Gorina E, Tusell JM. The orthopaedic status of severe haemophiliacs in Spain. Haemophilia 2000; 6: 170-6.
25 van Velzen AS, Eckhardt CL, Streefkerk N, Peters M, Hart DP, Hamulyak K, Klamroth R, Meijer K, Nijziel M, Schinco P, Yee TT, van der Bom JG, Fijnvandraat K. The incidence and treatment of bleeding episodes in non-severe haemophilia A patients with inhibitors. Thromb Haemost 2015; 115: 543-50.
26 Barrowcliffe TW, Raut S, Sands D, Hubbard AR. Coagulation and Chromogenic Assays of Factor VIII Activity: General Aspects, Standardization, and Recommendations. Semin Thromb Hemost 2002; 28: 247-56.
27 Mikaelsson M. Influence of phospholipids on the assessment of factor VIII activity. Haemophilia 1998; 4**.**
28 Mikaelsson M, Oswaldsson U. Assaying the Circulating Factor VIII Activity in Hemophilia A Patients Treated with Recombinant Factor VIII Products. Semin Thromb Hemost 2002; 28: 257-64.
29 Brummel-Ziedins KE, Branda RF, Butenas S, Mann KG. Discordant fibrin formation in hemophilia. J Thromb Haemost 2009; 7: 825-32.
30 Ragni M V., DiMichele DM, Hay CM, Malec LM, Seaman CD, Li J, Yabes JG, Butenas S, Brummel-Ziedins K. Thrombin generation and bleeding in haemophilia inhibitor patients during immune tolerance induction. Haemophilia 2016; 22: 240-7.
31 Saarela J, Rehn M, Oikarinen A, Autio-Harmainen H, Pihlajaniemi T. The short and long forms of type XVIII collagen show clear tissue specificities in their expression and location in basement membrane zones in humans. Am J Pathol. 1998 Aug;153(2):611-26.
32 Elamaa H, Snellman A, Rehn M, Autio-Harmainen H, Pihlajaniemi T. Characterization of the human type XVIII collagen gene and proteolytic processing and tissue location of the variant containing a frizzled motif. Matrix Biol. 2003 Sep;22(5):427-42.
33 Seppinen L, Pihlajaniemi T. The multiple functions of collagen XVIII in development and disease. Matrix Biol. 2011 Mar;30(2):83-92.
34 C.L. Bager, M.A. Karsdal. Biochemistry of Collagens, Laminins and Elastin, Chapter 18 - Type XVIII Collagen. 2016, 113-121, ISBN 9780128098479.

## Claims

1. An antibody specifically reactive with short isoform collagen type XVIII, wherein said antibody does not react with intermediate isoform collagen type XVIII or with long isoform collagen type XVIII, wherein the antibody is specifically reactive with the N-terminal amino acid sequence H₂N-EPERISEEVG (SEQ ID NO: 1),and wherein the antibody does not specifically recognise or bind an N-extended elongated version of said N-terminal amino acid sequence which is H₂N-AEPERISEEVG (SEQ ID NO: 3) and/or does not specifically recognise or bind an N-truncated version of said N-terminal amino acid sequence which is H₂N-PERISEEVG (SEQ ID NO: 4).

2. An antibody as claimed in any preceding claim, wherein the antibody is a monoclonal antibody.

3. A method of immunoassay for detecting or quantitating in a sample short isoform collagen type XVIII, wherein said method comprises contacting a sample comprising said short isoform collagen type XVIII with an antibody as claimed in claim 1 or 2, and determining the amount of binding of said antibody.

4. A method as claimed in claim 3, wherein the sample is a biofluid.

5. A method as claimed in claim 4, wherein the biofluid is serum, plasma, urine, cerebrospinal fluid, or amniotic fluid.

6. A method as claimed in any one of claims 3 to 5, wherein the immunoassay is a competition assay or a sandwich assay.

7. A method as claimed in any one of claims 3 to 6, wherein the immunoassay is a radioimmunoassay or an enzyme-linked immunosorbent assay.

8. A method as claimed in any one of claims 3 to 7, wherein the method further comprises correlating the quantity of said short isoform collagen type XVIII determined by said method with standard haemophilic disease samples of known disease severity to evaluate the severity of a haemophilic disease.

9. A method as claimed in any one of claims 3 to 8, wherein the method further comprises comparing the quantity of said short isoform collagen type XVIII determined by said method with standard values associated with healthy subjects to evaluate the presence and/or severity of a haemophilic disease.

10. A method as claimed in any one of claims 3 to 7, wherein the method further comprises quantifying the amount of collagen type XVIII in at least two samples obtained from a subject at a first time point and at at least one subsequent time point,
wherein an increase in the quantity of collagen type XVIII from the first time point to the at least one subsequent time point is indicative of a deterioration in a haemophilic disease from the first time point to the at least one subsequent time point, or
wherein a decrease in the quantity of collagen type XVIII from the first time point to the at least one subsequent time point is indicative of an improvement in a haemophilic disease from the first time point to the at least one subsequent time point.

11. A method as claimed in any one of claims 8 to 10, wherein the haemophilic disease may be haemophilic arthropathy.

12. A method for evaluating the efficacy of a drug for treating a haemophilic disease, wherein the method comprises using the method as claimed in any one of claims 3 to 7 to quantify the amount of collagen type XVIII in at least two samples obtained from a subject at a first time point and at at least one subsequent time point during a period of administration of the drug to the subject,
wherein a reduction in the quantity of collagen type XVIII from the first time point to the at least one subsequent time point during the period of administration of the drug is indicative of an efficacious drug for treating a haemophilic disease.

13. An assay kit for determining the quantity of short isoform collagen type XVIII, the kit comprising an antibody as claimed in claims 1 or 2 and at least one of:
- a streptavidin coated 96 well plate
- a peptide which is reactive with said antibody, which may be a biotinylated peptide H₂N-EPERISEEVG-L-Biotin (SEQ ID NO: 5), wherein L is an optional linker
- an optionally biotinylated secondary antibody for use in a sandwich immunoassay
- a calibrator peptide comprising the N-terminal sequence H₂N-EPERISEEVG (SEQ ID NO: 6)
- an antibody HRP labelling kit
- an antibody radiolabeling kit
- an assay visualization kit.

## Patentansprüche

1. Antikörper, der spezifisch mit der kurzen Isoform von Kollagen Typ XVIII reagiert, wobei der Antikörper nicht mit der intermediären Isoform von Kollagen Typ XVIII oder mit der langen Isoform von Kollagen Typ XVIII reagiert, wobei der Antikörper spezifisch mit der N-terminalen Aminosäuresequenz H₂N-EPERISEEVG (SEQ ID NO: 1) reagiert, und wobei der Antikörper eine N-erweiterte, verlängerte Version der N-terminalen Aminosäuresequenz, die H₂N-AEPERISEEVG (SEQ ID NO: 3) ist, nicht spezifisch erkennt oder bindet und/oder eine N-verkürzte Version der N-terminalen Aminosäuresequenz, die H₂N-PERISEEVG (SEQ ID NO: 4) ist, nicht spezifisch erkennt oder bindet.

2. Antikörper nach einem vorherigen Anspruch, wobei der Antikörper ein monoklonaler Antikörper ist.

3. Verfahren eines Immunassays zum Detektieren oder zur Quantifizieren einer kurzen Isoform von Kollagen Typ XVIII in einer Probe, wobei das Verfahren ein Inkontaktbringen einer Probe, die die kurze Isoform von Kollagen Typ XVIII umfasst, mit einem Antikörper nach Anspruch 1 oder 2 und ein Bestimmen der Bindungsmenge des Antikörpers umfasst.

4. Verfahren nach Anspruch 3, wobei die Probe ein Biofluid.

5. Verfahren nach Anspruch 4, wobei das Biofluid Serum, Plasma, Urin, Liquor oder Fruchtwasser ist.

6. Verfahren nach einem der Ansprüche 3 bis 5, wobei der Immunassay ein Kompetitionsassay oder ein Sandwichassay ist.

7. Verfahren nach einem der Ansprüche 3 bis 6, wobei der Immunassay ein Radioimmunoassay oder ein Enzym-Immunassay ist.

8. Verfahren nach einem der Ansprüche 3 bis 7, wobei das Verfahren ferner ein Korrelieren der durch das Verfahren bestimmten Menge der kurzen Isoform Kollagen Typ XVIII mit Standardproben hämophiler Erkrankung mit bekanntem Schweregrad der Erkrankung umfasst, um den Schweregrad einer hämophilen Erkrankung zu bewerten.

9. Verfahren nach einem der Ansprüche 3 bis 8, wobei das Verfahren ferner ein Vergleichen der durch das Verfahren bestimmten Menge der kurzen Isoform Kollagen Typ XVIII mit Standardwerten umfasst, die mit gesunden Subjekten assoziiert sind, um das Vorliegen und/oder den Schweregrad einer hämophilen Erkrankung zu bewerten.

10. Verfahren nach einem der Ansprüche 3 bis 7, wobei das Verfahren ferner ein Quantifizieren der Menge an Kollagen Typ XVIII in mindestens zwei Proben umfasst, die von einem Subjekt zu einem ersten Zeitpunkt und zu mindestens einem nachfolgenden Zeitpunkt erlangt werden,
wobei eine Zunahme der Menge an Kollagen Typ XVIII von dem ersten Zeitpunkt bis zu dem mindestens einen nachfolgenden Zeitpunkt indikativ für eine Verschlechterung einer hämophilen Erkrankung von dem ersten Zeitpunkt bis zu dem mindestens einen nachfolgenden Zeitpunkt ist, oder
wobei eine Abnahme der Menge an Kollagen Typ XVIII von dem ersten Zeitpunkt zu dem mindestens einen nachfolgenden Zeitpunkt indikativ für eine Verbesserung einer hämophilen Erkrankung von dem ersten Zeitpunkt zu dem mindestens einen nachfolgenden Zeitpunkt ist.

11. Verfahren nach einem der Ansprüche 8 bis 10, wobei die hämophile Erkrankung eine hämophile Arthropathie sein kann.

12. Verfahren zum Bewerten der Wirksamkeit eines Arzneimittels zum Behandeln einer hämophilen Erkrankung, wobei das Verfahren ein Verwenden des Verfahrens nach einem der Ansprüche 3 bis 7 umfasst, um die Menge an Kollagen Typ XVIII in mindestens zwei Proben zu quantifizieren, die von einem Subjekt zu einem ersten Zeitpunkt und zu mindestens einem nachfolgenden Zeitpunkt während eines Zeitraums einer Verabreichung des Arzneimittels an das Subjekt erlangt werden,
wobei eine Verringerung der Menge an Kollagen Typ XVIII von dem ersten Zeitpunkt bis zu dem mindestens einen nachfolgenden Zeitpunkt während des Zeitraums der Verabreichung des Arzneimittels indikativ für ein wirksames Arzneimittel zum Behandeln einer hämophilen Erkrankung ist.

13. Assay-Kit zum Bestimmen der Menge an kurzer Isoform Kollagen Typ XVIII, das Kit umfassend einen Antikörper nach Anspruch 1 oder 2 und mindestens eines von Folgenden:
- eine Streptavidin-beschichtete 96-Well-Platte
- ein Peptid, das mit dem Antikörper reagiert,
das ein biotinyliertes Peptid _{H2N-EPERISEEVG-L-Biotin} (SEQ ID NO: 5) sein kann, wobei L ein optionaler Linker ist,
- einen optional biotinylierten Sekundärantikörper zur Verwendung in einem Sandwich-Immunassay
- ein Kalibrierungspeptid umfassend die N-terminale Sequenz _{H2N-EPERISEEVG} (SEQ ID NO: 6)
- ein Antikörper-HRP-Markierungskit
- ein radioaktives Antikörper-Markierungskit
- ein Assay-Visualisierungskit.

## Revendications

1. Anticorps spécifiquement réactif avec le collagène isoforme court de type XVIII, dans lequel ledit anticorps ne réagit pas avec le collagène isoforme intermédiaire de type XVIII ou avec le collagène isoforme long de type XVIII, dans lequel l'anticorps est spécifiquement réactif avec la séquence d'acides aminés N-terminale H₂N-EPERISEEVG (SEQ ID NO : 1), et dans lequel l'anticorps ne reconnaît pas ou ne se lie spécifiquement pas à une version allongée N-étendue de ladite séquence d'acides aminés N-terminale qui est H₂N-AEPERISEEVG (SEQ ID NO : 3) et/ou ne reconnaît pas ou ne se lie pas spécifiquement à une version N-tronquée de ladite séquence d'acides aminés N-terminale qui est H₂N-PERISEEVG (SEQ ID NO : 4) .

2. Anticorps selon l'une quelconque des revendications précédentes, dans lequel l'anticorps est un anticorps monoclonal.

3. Procédé de dosage immunologique pour détecter ou quantifier dans un échantillon du collagène isoforme court de type XVIII, dans lequel ledit procédé comprend la mise en contact d'un échantillon comprenant ledit collagène isoforme court de type XVIII avec un anticorps selon la revendication 1 ou 2, et la détermination de la quantité de liaison dudit anticorps.

4. Procédé selon la revendication 3, dans lequel l'échantillon est un biofluide.

5. Procédé selon la revendication 4, dans lequel le biofluide est un sérum, plasma, urine, liquide céphalo-rachidien ou liquide amniotique.

6. Procédé selon l'une quelconque des revendications 3 à 5, dans lequel le dosage immunologique est un dosage par compétition ou un dosage en sandwich.

7. Procédé selon l'une quelconque des revendications 3 à 6, dans lequel le dosage immunologique est un dosage radio-immunologique ou un dosage d'immunoabsorption enzymatique.

8. Procédé selon l'une quelconque des revendications 3 à 7, dans lequel le procédé comprend en outre la corrélation de la quantité dudit collagène isoforme court de type XVIII déterminée par ledit procédé avec des échantillons de maladie hémophilique standard de gravité de maladie connue pour évaluer la gravité d'une maladie hémophilique.

9. Procédé selon l'une quelconque des revendications 3 à 8, dans lequel le procédé comprend en outre la comparaison de la quantité dudit collagène isoforme court de type XVIII déterminée par ledit procédé avec des valeurs standard associées à des sujets sains pour évaluer la présence et/ou la gravité d'une maladie hémophilique.

10. Procédé selon l'une quelconque des revendications 3 à 7, dans lequel le procédé comprend en outre la quantification de la quantité de collagène de type XVIII dans au moins deux échantillons obtenus d'un sujet à un premier point temporel et à au moins un point temporel ultérieur,
dans lequel une augmentation de la quantité de collagène de type XVIII du premier point temporel à l'au moins un point temporel ultérieur indique une aggravation d'une maladie hémophilique du premier point temporel à l'au moins un point temporel ultérieur, ou
dans lequel une diminution de la quantité de collagène de type XVIII du premier point temporel à l'au moins un point temporel ultérieur indique une amélioration d'une maladie hémophilique du premier point temporel à l'au moins un point temporel ultérieur.

11. Procédé selon l'une quelconque des revendications 8 à 10, dans lequel la maladie hémophilique peut être une arthropathie hémophilique.

12. Procédé d'évaluation de l'efficacité d'un médicament pour traiter une maladie hémophilique, dans lequel le procédé comprend l'utilisation du procédé selon l'une quelconque des revendications 3 à 7 pour quantifier la quantité de collagène de type XVIII dans au moins deux échantillons obtenus d'un sujet à un premier point temporel et à au moins un point temporel ultérieur pendant une période d'administration du médicament au sujet,
dans lequel une réduction de la quantité de collagène de type XVIII du premier point temporel à l'au moins un point temporel ultérieur pendant la période d'administration du médicament indique l'efficacité d'un médicament pour traiter une maladie hémophilique.

13. Kit de dosage pour déterminer la quantité de collagène isoforme court de type XVIII, le kit comprenant un anticorps selon les revendications 1 ou 2 et au moins l'un parmi :
- une plaque 96 puits revêtue de streptavidine,
- un peptide réactif avec ledit anticorps,
qui peut être un peptide H₂N-EPERISEEVG-L-Biotine (SEQ ID NO : 5) biotinylé, dans lequel L est un lieur facultatif
- un anticorps secondaire éventuellement biotinylé pour une utilisation dans un immunodosage en sandwich,
- un peptide calibrateur comprenant la séquence N-terminale H₂N-EPERISEEVG (SEQ ID NO : 6)
- un kit de marquage d'anticorps HRP
- un kit de radiomarquage d'anticorps
- un kit de visualisation de dosage.
